# EUROPEAN PATENT APPLICATION

(11) **EP 1 398 018 A1**
(43) Date of publication of application: **17.03.2004**
(21) Application number: 03445097.3
(22) Date of filing: 21.08.2003
(51) Int. Cl.: A61J 1/00

(54) **Container for dialysis fluid and a method for manufacturing of such container**

(30) Priority: 06.09.2002 SE 0202658
(71) Applicant: Gambro Lundia AB, 220 10 Lund (SE)
(72) Inventor: Axelsson, Mikael, 244 65 Furulund (SE); Bergqvist, Carin, 22655 Lund (SE)
(74) Representative: Larsson, Lena Eva-Karin

(57) **Abstract**

The invention relates to a container for containing a fluid that is to be introduced into or drained from a human body in peritoneal dialysis. The container comprises two opposite sidewalls (10, 11) of a flexible, plastic material. At least one of said sidewalls (10) is over its major part embossed with a pattern (12), which pattern is such that adhesion of the at least one sidewall (10) is prevented, and which pattern includes at least one zone (12b, 12b') having an embossed graduation (16, 17, 16', 17') indicative of the amount of fluid contained in the container.

The invention also relates to a method for manufacturing a container, use of a container and a kit of parts for peritoneal dialysis.

## Description

### Technical Field of the Invention

The present invention relates to a container for containing a dialysis fluid and/or dialysate, to a method for manufacturing of such a container, and further to the use of said container. Additionally, the invention relates to a kit comprising such a container.

### Background of the Invention

Dialysis is a well-known method for treatment of kidney insufficiency. Dialysis can be extracorporal or intracorporal. Peritoneal dialysis is an intracorporal method which includes exchanging solutes and water in capillary vessels of the patient's peritoneum for hypertonic solution. The peritoneum is used as a dialysis membrane.

In the most common form of peritoneal dialysis, known as CAPD (Continuous Ambulatory Peritoneal Dialysis), the patient manually changes the dialysis fluid every few hours.

The dialysis fluid or solution to be used in a patient is contained in a container or bag. The dialysis fluid flows through a line and a catheter (which has been inserted into the abdomen during an operation) into the peritoneal cavity, where the dialysis takes place - excess water and waste products from the blood are drawn across the peritoneum into the dialysis fluid. The dialysate (i.e. the used dialysis fluid) is then drained out and collected in a similar container or in the one previously containing the dialysis fluid. This used fluid, dialysate, is replaced with new, dialysis fluid for further dialysis.

To be able to overview a peritoneal dialysis treatment it is important that it is possible for the user or patient to determine the volume of the fluid let into the abdomen or which have been drained from the abdomen in an accurate and convenient manner.

Containers for containing a dialysis fluid, which are made of PVC and provided with a printed graduation indicative of the volume of fluid contained in the container, are available on the market.

There are however some disadvantages with containers according to prior art.

A graduation or scale that is printed on the outside of a container might end up slantingly on the container resulting in less accurate or erroneous readings. Furthermore, the printed graduation may fade and thus become less distinct, or smear and dislocate due to handling of the container, which also results in inadequate readings.

Additionally, there is a risk of that the printed graduation may cause migration of color or ink into the container material and the fluid contained within the container. This is as a matter of course highly undesirable as regards a container containing sterile dialysis solution. However, this is also true for drain containers in the case the drained fluid is to be analyzed.

Moreover, the manufacturing process for printed containers is rather costly due to the cost of printing color or ink and to the fact that the printing step is time consuming.

Another usual problem with known containers is that the sidewalls made of a flexible material composing the container often adhere or stick together. Furthermore, a container as such may stick together with other containers or a possible cover while being stored and transported.

In WO 93/14735 a manufacturing method for medical plastic containers with non-sticking interior walls is shown. The container sheets are formed in double layers, whereby the interior layer is formed with a high-temperature gel which is gelled at a temperature higher than the gelling temperature of the exterior layer composite, and a fine embossing on the interior surface is created.

With regards to the choice of material, there is a growing global desire from health authorities to replace products in PVC for products made of non-PVC materials.

### Summary of the Invention

In these circumstances it is an object of the present invention to provide an improved container to be used in peritoneal dialysis and a method for manufacturing of such a container.

This and other objects are achieved by a container comprising the features of the enclosed independent device claim and a method comprising the steps of the enclosed independent method claim. Preferred embodiments are set forth in the enclosed dependent claims and in the following description.

By having at least one of the sidewalls of the container provided with an embossed pattern over its major part the tendency of the wall to stick or adhere to adjacent surfaces is reduced. The providing of an embossed graduation alleviates or eliminates the problems of and disadvantages with for example a printed graduation according to prior art, such as slant positioning, fading, smearing, color migration etc. The volume readings will become more reliable.

According to the invention, the dense pattern preventing adhesion and the graduation are both provided by means of embossing and on the same at least one sidewall. These facts and the fact that the graduation is a part or a zone of the embossed pattern facilitate the manufacturing process of such containers, which process by that means becomes faster and more cost-effective.

An embossed pattern that, according to a preferred embodiment of the inventive container, protrudes on both sides of the at least one sidewall yields a sidewall that does not adhere to either the other sidewall of the container or to any other adjacent surface or objects. This is an advantage since it is desirable to be able to packet a container containing dialysis fluid in a cover or together with an empty drain container, the two containers being interconnected with a tube or line set, and wrap the whole kit up in a cover.

It is preferred to let the other sidewall of the container be transparent to allow taking of a reading of the amount of fluid in the container. The transparency also makes it easier for the patient/user to check for signs of peritonitis.

The method according to the invention for manufacturing of an inventive container is fast and cost-effective. The method makes use of an embossing tool. The advantage of letting the tool comprise a first and second element, the elements having a pattern corresponding to the pattern of the completed container, wherein the second element corresponds to the pattern of said zone, is that in case another graduation is desirable, just one element and not the whole tool need be exchanged.

### Brief Description of the Drawings

Fig 1 illustrates the principle of the use of one embodiment of the inventive container in peritoneal dialysis.
Fig 2 shows a container for dialysate according to a preferred embodiment of the invention.
Fig 3 illustrates the principle of the use of another preferred embodiment of the inventive container.
Fig 4 shows a container adapted for containing either sterile dialysis fluid or dialysate according to another preferred embodiment of the invention.

### Detailed Description of Preferred Embodiments

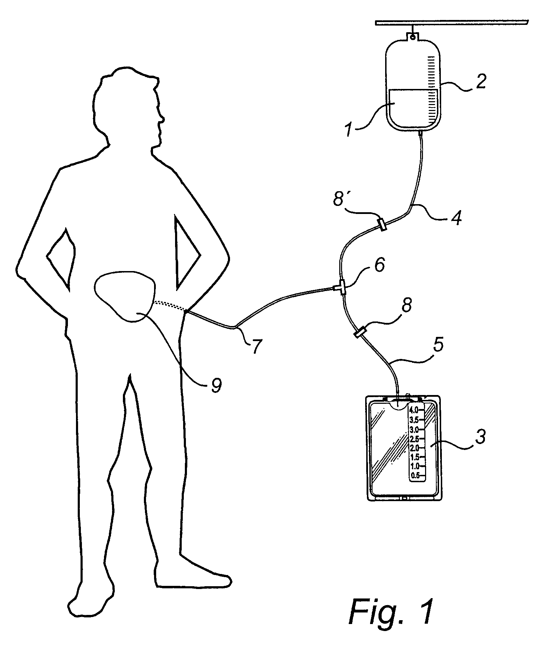

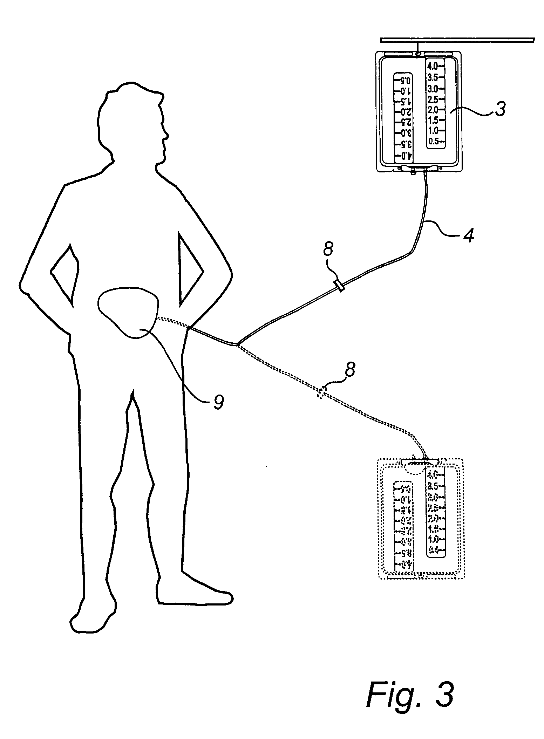

In peritoneal dialysis, see fig 1 and 3, the peritoneal cavity is utilized as artificial kidney. In fig 1 a dialysis solution 1 to be introduced into the patient's body is contained in a first container 2. The first container 2 is interconnected with a second container 3 via a line set comprising first and second lines 4, 5 jointed by means of a y- or t-piece 6. In use, the third leg of the coupling 6 is connected to the catheter 7 which has been inserted to the patient's abdomen. The first line 4 is clamped by a hose clamp or clip 8' and the dialysate 1 is drained from the peritoneal cavity 9 by placing the drain bag 3 at a level such that the dialysate may drain by means of gravity through the catheter 7 and the line 5. The clamp 8' is removed. The second line 5 on the drainage side is clamped by a clamp 8. The first container 2 is positioned in such a manner so that the dialysis fluid or solution 1 may be introduced via the line 4 and the catheter 7 into the peritoneal cavity 9 by the force of gravity. The peritoneal cavity 9 is flooded with the solution and the solution is left within the cavity 9 for an appropriate lapse of time. Removal of solutes and excess water takes place across the peritoneum which acts as a semi permeable membrane.

Hereinafter a preferred embodiment of a drain bag 3 according to the invention will be described. It is easily appreciated that the inventive idea can be applied to containers for containing drained fluid, dialysate, as well as containers for containing clean dialysis solution to be introduced into a patient.

A solution container and a drain container may be packed together as a kit including a line set and clamps and wrapped up in a cover. Alternatively, a single solution container including a line set may be wrapped up in a cover.

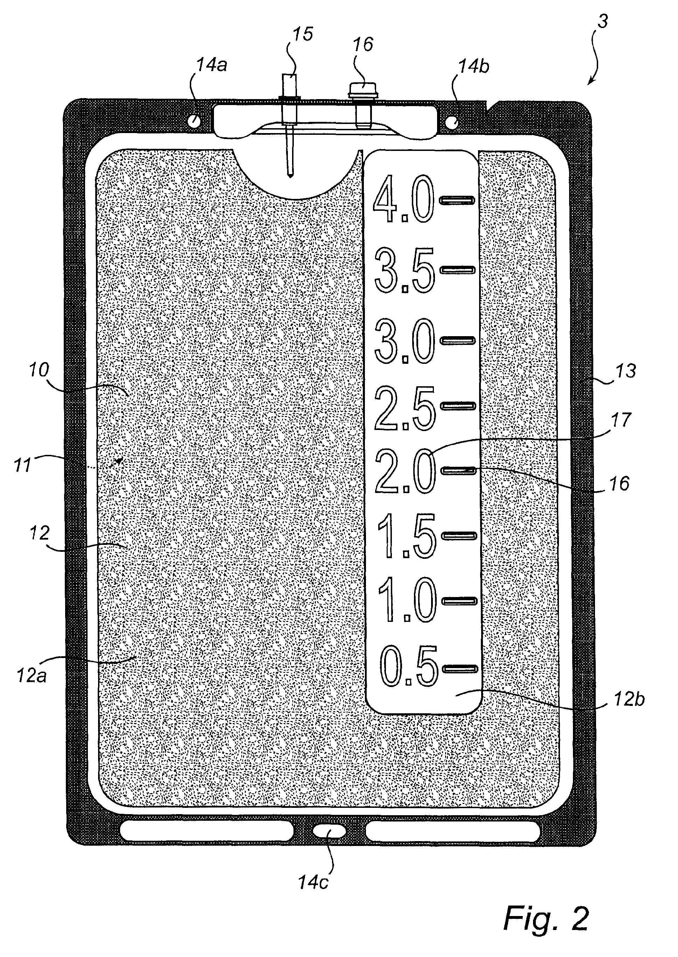

The drain container 3 in fig 2 comprises two opposite sidewalls 10, 11 made of a flexible, transparent, preferably non-PVC, plastic material, such as PA/PP (polyamide/polypropylene). The first wall 10 is provided with an embossed pattern 12 over its major part. The pattern 12 comprises a first zone 12a and a second zone 12b. The first zone 12a has a dense pattern with embossments. By embossments is meant bosses and dents. The bosses protrude on either side of the wall 10. That is, both the inner and outer surface of the sidewall 10 is provided with bosses and dents. With dense pattern is meant that the bosses are sufficiently frequent or large to make the surface less inclined to adhesion to adjacent surfaces. The second zone 12b includes an embossed graduation indicative of the volume of fluid possibly contained in the container. The second sidewall 11 is transparent and have no embossments, which allows the taking of readings by looking through this wall 11.

The first 10 and second 11 sidewalls are welded together along their periphery. The weld 13 is at the upper and lower ends of the bag provided with holes 14a-c for suspension purposes. In the upper part two valves 15, 16 are arranged, of which one valve 15 is adapted to be connected to the line set through which fluid is allowed to pour into or out of the bag. Optionally, a desirable substance may be introduced into the container through the second valve 16.

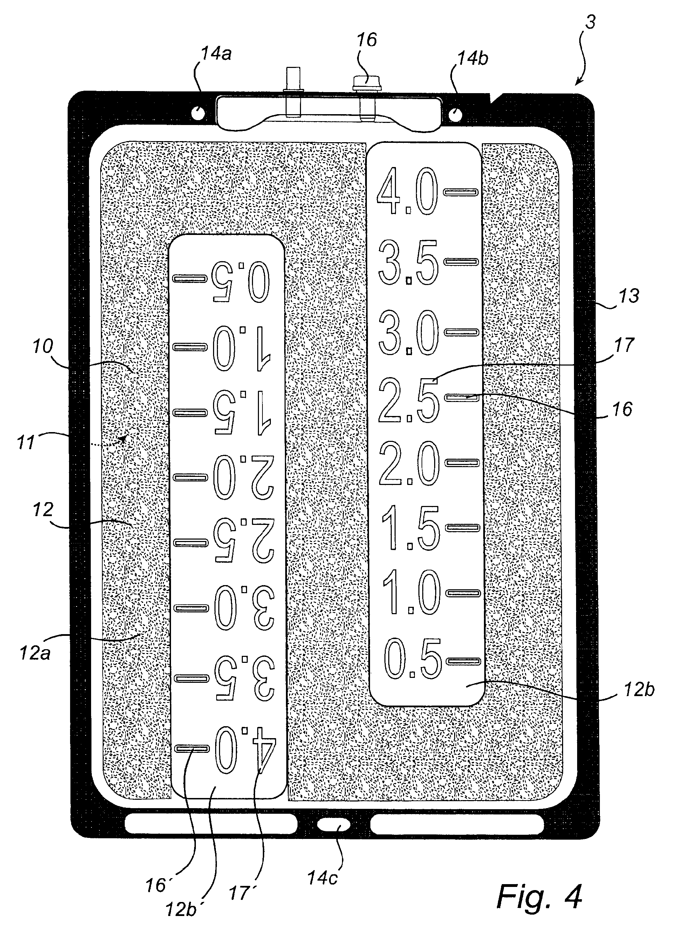

An alternative embodiment of the invention is shown in fig 3 and 4. In fig 3 is shown that an inventive container may be used for both sterile dialysis solution and dialysate. Preferably, the container is provided with two volume scales, see fig 4. First, the container containing dialysis solution is arranged at a level allowing the solution to flow into the patient's peritoneal cavity 9. After a certain time, the bag is turned "upside-down" and lowered to a level appropriate for drainage of the dialysate from the cavity 9.

The scale or graduation comprises graduations or graduation lines 16, 16' and numerals 17, 17' and is indicative of volume. The maximum volume which can be contained in a container according to this embodiment of the invention is about 3.8 litres. Normally, containers are adapted to hold 1 to 6 litres, usually 2 to 2.5 litres.

It is appreciated that the placement of the graduations according to fig 2 and 4 are shown as examples and that the graduations may be located anywhere on the surface of the one or both sidewalls.

It is further appreciated that the container, especially a container for containing clean solutions, could be of multi-compartment type wherein one or more compartments are provided with an embossed pattern according to the above.

An inventive container may be manufactured according to the following. A film of a flexible, preferably non-PVC material is arranged close to an embossing tool comprising a first and a second element. The first element is provided with a pattern corresponding to the pattern of the first zone 12a, 12a' of the completed container. The at least one second element has a pattern corresponding to the pattern of the at least one second zone 12b, 12b', comprising the graduation 16, 16', 17, 17' of the completed container. Then the film is heated and brought into contact with the tool by means of a vacuum, whereby the pattern and the graduation(s) are created in the film. The film is arranged at the tool until the embossed pattern remain permanent. The film is cut to the form of the first sidewall 10 of the container. This first sidewall 10 and a second sidewall 11 are joined together along their periphery by means of welding. The latter step includes the arranging of the above-mentioned valves in the welding seam. Suitable process variables, such as temperature, will be easily determined by means of trial and error by a person skilled in the art.

The inventive method provides a time and cost effective manufacturing process. Alternatively, the pattern could also be embossed by rolling of a continuous plastic film, whereby the lateral area of the roll has a pattern corresponding to the desired pattern including one or more volume graduations of the container.

## Claims

1. A container for containing a fluid that is to be introduced into or drained from a human body in peritoneal dialysis, comprising two opposite sidewalls (10, 11) of a flexible, plastic material,
**characterized in that** at least one of said sidewalls (10) over its major part being embossed with a pattern (12), which pattern being such that adhesion of the at least one sidewall (10) being prevented, and which pattern includes at least one zone (12b, 12b') having an embossed graduation (16, 17, 16', 17') indicative of the amount of fluid contained in the container.

2. A container according to claim 1, wherein said material is a non-PVC material.

3. A container according to claim 1 or 2, wherein the embossed pattern (12) protrudes on both sides of said at least one sidewall (10) .

4. A container according to any one of claims 1-3, wherein the other sidewall (11) is transparent to allow taking of a reading of the graduation (16, 17, 16', 17').

5. A container according to any one of claims 1-4, wherein said at least one graduation (16, 17, 16', 17') is indicative of the volume of the fluid contained in the container.

6. A container according to any one of claims 1-5, being provided with two mutually inverted graduations (16, 17, 16' , 17').

7. A method for manufacturing a container according to any one of claims 1-6, comprising the steps of:
- arranging a film of a flexible, plastic material at a tool comprising a first and at least one second element, the elements having a pattern corresponding to the pattern (12) of the completed container, wherein the at least one second element corresponds to the pattern of said at least one zone (12b, 12b'),
- heating the film,
- bringing the film into contact with the tool, whereby the pattern (12) is created in the film,
- the film being arranged at the tooluntil the embossed pattern (12) remain permanent,
- cutting the film to constitute the first sidewall (10) of the container,
- joining the first sidewall (10) with the second sidewall (11) to form the container.

8. A method according to claim 7, wherein the step of joining the first (10) and second (11) sidewalls comprises welding of the sidewalls together along their peripheries.

9. Use of a container according to any one of claims 1-6 in peritoneal dialysis, the container containing a solution to be introduced into a patient's peritoneal cavity.

10. Use of a container according to any one of claims 1-6 in peritoneal analysis, the container being adapted to receive dialysate drained from a patient's peritoneal cavity.

11. A kit of parts for peritoneal dialysis comprising
- a first flexible, plastic container adapted for containing a dialysis fluid to be introduced into the peritoneal cavity of a patient,
- a second flexible, plastic container adapted for receiving fluid drained from the peritoneal cavity,
- a set of lines connecting the two containers via a coupling,
- at least one clamping device adapted to clamp one of said lines,
**characterized in that** at least one of the first and second containers is a container according to any one of claims 1-5.

12. A kit of parts for peritoneal dialysis comprising
- a flexible, plastic container adapted for containing a dialysis fluid to be introduced into the peritoneal cavity of a patient, as well as for receiving dialysate drained from the peritoneal cavity,
- a coupling,
- a line connecting the container and the coupling, and
- a clamping device adapted to clamp said line,
**characterized in that** the container is a container according to any one of claims 1-6.
